# EUROPEAN PATENT SPECIFICATION

(11) **EP 2 952 182 B1**
(45) Date of publication and mention of the grant of the patent: **23.11.2022**
(21) Application number: 14171153.1
(22) Date of filing: 04.06.2014
(51) Int. Cl.: A61K 31/00, A61K 31/7068, A61P 31/12, A61P 37/04

(54) **Compounds and methods for increasing the immune response to papillomavirus**
Verbindungen und Verfahren zur Erhöhung der Immunreaktion gegen Papillomavirus
Composés et procédés pour augmenter la réponse immunitaire au virus du papillome

(43) Date of publication of application: 09.12.2015
(73) Proprietor: Universität Heidelberg, 69117 Heidelberg (DE)
(72) Inventor: Von Knebel Doeberitz, Magnus, 69118 Heidelberg (DE); Vinokurova, Svetlana, 115478 Moscow (RU); Reuschenbach, Miriam, 81829 München (DE); Sauer, Madeleine, 40699 Erkrath (DE)
(74) Representative: Altmann Stößel Dick Patentanwälte PartG mbB

(56) References cited:
- WO-A1-00/33832
- WO-A1-2005/115450
- WO-A1-2010/004251
- WO-A2-2009/062604
- LI HUILI ET AL: "Immune regulation by low doses of the DNA methyltransferase inhibitor 5-azacitidine in common human epithelial cancers", ONCOTARGET, IMPACT JOURNALS LLC, UNITED STATES, vol. 5, no. 3, 1 January 2014 (2014-01-01) , pages 587-598, XP009181100, ISSN: 1949-2553
- KALANTARI M ET AL: "Effects of cellular differentiation, chromosomal integration and 5-aza-2'-deoxycytidine treatment on human papillomavirus-16 DNA methylation in cultured cell lines", VIROLOGY, ELSEVIER, AMSTERDAM, NL, vol. 374, no. 2, 10 May 2008 (2008-05-10), pages 292-303, XP022632716, ISSN: 0042-6822, DOI: 10.1016/J.VIROL.2007.12.016 [retrieved on 2008-02-01]
- LU D ET AL: "Treatment with demethylating agent, 5-aza-2'-deoxycytidine enhances therapeutic HPV DNA vaccine potency", VACCINE, ELSEVIER, AMSTERDAM, NL, vol. 27, no. 32, 9 July 2009 (2009-07-09), pages 4363-4369, XP026210586, ISSN: 0264-410X, DOI: 10.1016/J.VACCINE.2009.02.041 [retrieved on 2009-02-24]

## Description

The present disclosure relates to a DNA demethylating agent and to a pharmaceutical composition comprising said DNA demethylating agent and at least a pharmaceutical carrier for use in increasing the immune response to papillomavirus (PV) of a subject infected with PV. Further, the present disclosure relates to the use of a DNA demethylating agent for increasing the immune response to PV of a subject infected with PV. The present disclosure also relates to a method for increasing the immune response to a PV of a subject infected with said PV, comprising contacting PV-infected cells of said subject infected with PV with a DNA demethylating agent, and, thereby, increasing the immune response to PV of said subject infected with PV. Moreover, the present disclosure relates to a method of killing a PV-infected cell, comprising contacting said PV-infected cell with a DNA demethylating agent, contacting said PV-infected cell with at least one PV antigen specific T-cell, and, thereby, killing said PV-infected cell.

DNA methylation has been known as the biochemical process of adding a methyl group to a cytosine or an adenosine residue comprised in DNA. In mammals, typically, the cytosine residues in CpG dinucleotides are methylated by the action of one of several DNA cytosine methyltransferase enzymes.

DNA methylation was found to have a profound impact on gene expression, with expression of a specific gene decreasing with an increase of the number of methylated CpG dinucleotides in the vicinity of said gene and its promoter. Accordingly, DNA methylation has been identified as an important regulatory mechanism in the control of gene expression, in particular in development of metazoan organisms. However, DNA methylation was also identified as an important factor in carcinogenesis and in the cellular defense against viruses. Accordingly, Li et al. (Oncotarget 5(3): 587 (2014)) established that low-dose treatment with 5-azacytidine leads to expression of immune-related genes in cancer cell lines.

Papillomaviruses (PVs) are a group of dsDNA viruses infecting all vertebrate species. Typically, PVs infect the basal cells of squamous epithelial tissues. Most infections seem to end in an abortive state as the viral genomes appear to be silenced by extensive epigenetic methylation of the viral genomes (Vinokurova and von Knebel Doeberitz, M. (2011). PloS one 6, e24451), von Knebel Doeberitz and Vinokurova, S. (2009). Archives of medical research 40, 435-442). In case that the HPV-infection triggers outgrowth of epithelial lesions, few HPV genomes within a given cell appear not to be fully silenced and may start to express HPV early genes including the E6 and E7 genes that lead to proliferation of the affected basal / parabasal cells. If these cells in frame of the normal differentiation pathway start to differentiate and are pushed upwards into the intermediate cell layers, they modify their cellular epigenetic milieu that also impacts the methylation pattern of HPV-genomes (Vinokurova and von Knebel Doeberitz, 2011, loc. cit.). This results in substantially enhanced expression of the viral early genes and replication of the viral genomes triggered by the viral E1 and E2 gene products. Finally, if the cells reach in frame of the normal differentiation modus the superficial cells of the squamous epithelium, they again shift the epigenetic milieu. This results in a complete reprogramming of the viral genomes: the early genes are now silenced, however the late genes, in particular the L2 and L1 become activated due to demethylation of the respective CpG di-nucleotides of the HPV-genome.

Typically, PVs induce benign hyperproliferative lesions. However, some PV-types cause the intraepithelial lesions that may progress to high grade pre-neoplastic or even fully malignant lesions. These PVs were classified as agents carcinogenic to humans by WHO's International Agency for Research on Cancer (IARC) and are referred to as high risk HPV types (HR-HPVs). HR-HPV types encode two potent viral oncogenes referred to as E6 and E7 that are required to initiate and maintain the neoplastic features of the infected and transformed host cell. The biochemical processes leading to malignant transformation rely on the active expression of these genes in the basal and parabasal squamous epithelial cells. HPV-related cancers almost exclusively arise at distinct anatomical areas as e.g. the transformation zone of the uterine cervix in women, the linea dentata in case of anal lesions or the Waldeyer epithelium in the oropharynx. The squamous epithelium at these sites apparently display a highly specific gene expression profiles (Herfs et al. (2012). PNAS 109, 10516-10521), suggesting that they are derived from embryonic remnants.

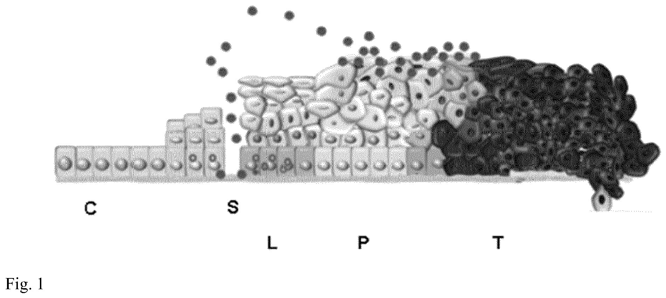

The highly restricted expression of HPV genes in differentiating squamous epithelial cells allows HPVs to hide from the immune system. Due to the very low levels of the viral genes expressed in the initially infected basal cell compartment, there are virtually no viral antigens exposed to the immune system of the infected host. Only if these cells have undergone terminal differentiation and irreversible senescence, HPV genes start to be more actively expressed. Importantly, expression of the highly immunogenic genes L1 and L2 is restricted to the very superficial squamous epithelial cells that lack antigen presentation capacities (see Fig. 1). Thereby, PVs virtually avoid contact with cells that could trigger a spontaneous immune response. This phenomenon explains why it usually takes weeks or even months until a robust immune response builds up in the HPV-infected host that finally may induce regression of the HPV-infected lesions.

WO 2009/062604 A2 reports compounds and methods useful in detection and therapy of HPV-associated diseases. WO 2010/004251 A1 teaches methods and kits for diagnosing and/or monitoring the progression of or otherwise staging a disease caused by an infection by a double stranded DNA virus in a test sample obtained from a subject comprising determining the methylation status of the viral genome. WO 00/33832 A1 relates to tea catechin as a major chemical component, demonstrating that tea catechin when applied to human papilloma virus associated with cervical intraepithelial neoplasia I, II, III, and IV including chronic cervicitis are either preventable from further progression of disease state and/or cured completely. Lu et al. (2009), Vaccine 27:4363 reported that treatment with the demethylating agent 5-aza-2'-deoxycytidine enhances therapeutic HPV DNA vaccine potency.

Thus, there is a substantial need in the art for improving the immune response of an individual infected with PV in order to potentially eliminate the infection. The invention is set out in the appended set of claims.

Any references in the description to methods of treatment refer to the compounds, pharmaceutical compositions and medicaments of the present invention for use in a method for treatment of the human (or animal) body by therapy (or for diagnosis).

Accordingly, the present invention relates to a DNA demethylating agent for use according to claim 1; further disclosure relates to a DNA demethylating agent for use in improving the immune response to a papillomavirus (PV) of a subject infected with said PV.

As used herein, the term "DNA demethylating agent" relates to a chemical compound having the activity of causing DNA demethylation, i.e. the loss of methyl groups from nucleobases comprised within DNA. Thus, preferably, the DNA demethylating agent of the present invention is a compound causing, when contacted to a host cell at an effective concentration, the degree of methylation of the DNA comprised in said cell to decrease. More preferably, the DNA demethylating agent is an agent causing, when contacted to a host cell at an effective concentration, the degree of methylation of cytosine residues in CpG sites of the DNA comprised in said cell to decrease. Most preferably, the DNA demethylating agent is an agent causing, when contacted to a host cell infected with a PV at an effective concentration, the degree of methylation of Cytosine residues in CpG sites of the late promoter of said PV to decrease. In the context of the term "decrease of the degree of methylation", the term "degree of methylation" relates to the ratio of the number of methylated nucleobases, preferably 5-Methylcytosine residues, within a specific DNA subsequence, to the total number of (i.e. methylated and unmethylated) nucleobases, preferably Cytosine residues, within said specific DNA subsequence; the term decrease, accordingly, relates to a decrease of the value of the aforesaid ratio. Preferably, said ratio decreases in the presence of an effective concentration of the DNA demethylating agent by at least 5% of its value per cell division, more preferably by at least 10% of its value per generation, most preferably by at least 20% of its value per generation. Methods of determining the number of methylated nucleobases and the number of unmethylated nucleobases are known in the art. More preferably, the DNA demethylating activity of an agent is identified by incubating Caski cells in the presence of said agent for 28 days, followed by determining expression of the HPV L1 gene, wherein an increase of the HPV L1 gene as compared to control Caski cells incubated in the absence of said agent indicates DNA demethylating activity of said agent; even more preferably, incubation conditions and mode of detection of HPV L1 gene expression are selected as described in the Examples. Most preferably, an increase of L1 gene expression by a factor of at least 5 as compared to cells treated with vehicle alone is indicative of a DNA demethylating agent. Also preferably, the agent is used at a concentration of 1 µM in the assay or, in case the agent is toxic to Caski cells at the aforesaid concentration, at the highest non-toxic concentration usable.

In a preferred embodiment, the DNA demethylating agent is a chemical compound having the activity of causing DNA demethylation at CpG sites of said DNA. Accordingly, the degree of methylation preferably is determined by standard methods of molecular, e.g., preferably by bisulfite sequencing, more preferably bisulfite pyrosequencing.

Preferably, the DNA demethylating agent is an inhibitor of at least one DNA methyltransferase. DNA methyltransferases are well known in the art and, preferably, fall under one of EC classes 2.1.172, 2.1.1.113, or 2.1.1.37. More preferably, the DNA demethylating agent is an inhibitor of at least one DNA (cytosine-5-)-methyltransferase (EC 2.1.1.37). Even more preferably, the DNA demethylating agent is an inhibitor of a mammalian DNA methyltransferase (DNMT), most preferably of DNMT1. Assays for measuring the activity of a DNMT are known in the art and are commercially available, e.g. the DNMT Activity / Inhibition Assay marketed by ActiveMotif^{®}.

The DNA demethylating agent may be a non-covalent inhibitor of DNMT activity or, more preferably, a covalent inhibitor of DNMT activity. Preferably, the DNA demethylating agent is a non-covalent inhibitor of at least one DNA methyltransferase. Accordingly, more preferably, the DNA demethylating agent is a chemical compound inhibiting the activity of human DNA methyltransferase 1 (DNMT1) in vitro by at least 20% when present at a concentration of 1 µM in the assay mixture. Most preferably, the DNA demethylating agent is selected from the list consisting of 1-hydrazinylphthalazine (Hydralazine), 4-amino-N-(2-diethylaminoethyl) benzamide (Procainamide); 2-(diethylamino)ethyl 4-aminobenzoate (Procaine); 2-(1H)-pyrimidinone riboside (Zebularine); 2-amino-4-([[(2S,3S,4R,5R)-5-(6-amino-2-R1-9H-purin-9-yl)-3,4-dihydroxytetrahydrofuran-2-yl]methyl]sulfanyl)butanoic acid with R1 being chloro-, fluoro-, iodo-, methoxy-, methyl- or methylsulfanyl-; S-Tubercidinylhomocysteine; S-(N-(2-biphenyl-4-ylethyl)adenosyl)-L-homocysteine; S-(N-(2-biphenyl-4-ylethyl)-2-chloroadenosyl)-L-homocysteine; S-(N-phenylpropyladenosyl)-L-homocysteine; 8-aza-S-adenosyl-L-homocysteine; S-(N-(3,5-dimethoxybenzyl)adenosyl)-L-homocysteine; S-(N-(pyridin-4-ylmethyl)adenosyl)-L-homocysteine; S-(N-phenylethyladenosyl)-L-homocysteine; S-nebularinehomocysteine; S-(N-(2-biphenyl-4-ylethyl)-2-chloroadenosyl)-L-homocysteine; 1-deaza-S-adenosyl-L-homocysteine; 3-deaza-S-adenosyl-L-homocysteine; S-(1-deazaadenosyl)-L-homocysteine; S-(N-benzyladenosyl)-L-homocysteine; S-(N-phenyladenosyl)-L-homocysteine; and 1,2-dihydropyrimidin-2-one-5-methylene-(methylsolfonium)-adenosyl.

More preferably, the DNA demethylating agent is a covalent inhibitor of at least one DNA methyltransferase. Accordingly, even more preferably, the DNA demethylating agent is a chemical compound causing the measurable specific activity of human DNA methyltransferase (DNMT) in cell extracts from MCF-7 cells to decrease by at least 10% when present in the culture medium at a concentration of 1 µM for 5 days. Even more preferably, the DNA demethylating agent is selected from the list consisting of 5-Azacytidine, 5-Aza-2'-deoxycytidine, Arabinosyl-5-azacytidine, 5-6-Dihydro-5-azacytidine, 5-Fluoro-2'-deoxycytidine, and Epigallocatechin-3-gallate. Most preferably, the DNA demethylating agent is selected from the list consisting of 5-Azacytidine, 5-Aza-2'-deoxycytidine, and Arabinosyl-5-azacytidine.

The term "papillomavirus" or PV relates to members of the family of non-enveloped, dsDNA viruses generally known as Papillomaviridae infecting a subject of the present invention. Preferably, the term relates to PV of farm and/or companion animals, e.g. equine PV, canine PV, feline PV, PV of the pig, or bovine PV (BPV), more preferably to BPV-1, BPV-2, BPV-3, BPV-4, BPV-5, BPV-6, BPV-7, BPV-8, BPV-9, BPV-10, and Bovine alimentary Papilloma Virus-11 (BAPV-11). Also preferably, the term relates to a PV infecting humans (human PV, HPV). More preferably, the PV is a genotype infecting the human mucosa and associated with cancer, i.e. a high-risk (HR)-HPV or a putative HR-HPV. Even more preferably, the putative high-risk genotype is selected from the list consisting of HPV 26, 53, 67, 70, 73 and 82 or the high-risk HPV genotype is selected from the list consisting of the HR-HPV genotypes 16, 18, 31, 33, 35, 39, 45, 51, 52, 56, 58, 59, 68, 73 and 82. Most preferably, the PV is HPV 16 or 18.

As used herein, the term "PV positive" relates to the property of a cell or of a higher-order cellular structure, including, preferably, a tumor, a tissue, a lesion, or a subject, of being or having been infected with at least one PV. Preferably, a PV positive cell, group of cells, tumor, tissue, or subject is a cell, group of cells, tumor, tissue, or subject wherein at least one PV-specific antigen, more preferably PV-specific protein, most preferably PV L1 and/or L2 protein is detectable. More preferably, a PV positive cell, group of cells, tumor, tissue, or subject is a cell, group of cells, tumor, tissue, or subject wherein at least one PV-specific polynucleotide, more preferably PV L1 and/or L2 gene or an expressible fragment thereof is detectable.

The term "subject", as used in this specification, relates to an animal, preferably a mammalian animal, including, e.g., rat and mouse. More preferably, the subject is a companion or farm animal, including, e.g. cattle, horse, pig, sheep, cat, and dog. Most preferably, the subject is a human. The subject of the present invention is infected with PV, i.e., preferably, the subject comprises at least one cell or, more preferably, at least one group of cells infected with PV. More preferably, the subject comprises at least one distinguishable group of cells infected with PV. Most preferably, the subject is afflicted with at least one PV related lesion or/and with at least one PV-positive cancer.

The term "PV related lesion", as used herein, relates to a group of non-cancerous, PV-infected cells. Preferably, the term relates to a non-cancerous tumor caused by or associated with PV-infection of at least a fraction of the cells forming said tumor. Preferably, the PV related lesion is a PV-positive lesion selected from the list consisting of warts, exophytic growing papillomas, condylomata, inverted papillomas, any kind of HPV-induced squamous intraepithelial lesions (SIL), other pre-neoplastic or neoplastic PV-induced lesions, cervical neoplasias, skin neoplasias, neoplasias of the anal epithelium, neoplasias of the head and neck region, preferably of the oropharynx, or neoplasias of the tonsils.

As used herein, the term "PV-related cancer" relates to a cancerous tumor caused by or associated with PV-infection of at least a fraction of the cells forming said tumor. Preferably, the PV related cancer is a PV-positive tumor selected from the list consisting of cervical cancer, vulvar cancer, vaginal cancer, penile cancer, anal cancer, and head and neck cancer. Preferably head and neck cancer is oropharyngeal cancer.

As used herein, the term "host cell" or "cell" relates to an animal, more preferably to a mammalian cell, including, e.g. a rat and a mouse cell, even more preferably to a cell from a companion or farm animal, including, e.g. a cattle, horse, pig, sheep, cat, and dog cell, and, most preferably, to a human cell. Preferably, the cell is a cell infected with PV.

The term "immune response", as used herein, relates to any protective response of the body of a subject to an antigen involving activity of at least one type of lymphocyte and/or of at least one antigen-recognizing macromolecule. Preferably, the immune response comprises deactivation of said antigen by production of antigen-specific antibodies (humoral immune response). More preferably, the immune response comprises lysis of foreign cells or of body cells presenting said antigen (cell-mediated immune response).

Accordingly, the term "improving the immune response" relates to inducing an increase in the quality or intensity of the immune response of a subject to PV. Preferably, improving the immune response includes or is increasing expression of a PV-specific antigen or epitope, preferably of PV L1 and/or L2, in a cell expressing at least one of MHC class I and MHC class II. More preferably, improving the immune response includes or is increasing expression of a PV-specific antigen or epitope, preferably of PV L1 and/or L2, in a cell of the skin, most preferably a basal cell. Also preferably, improving the immune response includes or is increasing the frequency of PV antigen-specific T-cells in the blood of a subject. More preferably, improving the immune response is increasing the frequency of PV antigen-specific CD-4+ and CD-8+ T-cells in the blood of said subject. Most preferably, improving the immune response is increasing the frequency of activated PV antigen-specific CD-4+ and CD-8+ T-cells in the blood of said subject. Accordingly, improving the immune response preferably comprises activating the expression of immunogenic viral gene products in cells of the basal and para-basal cell layers of an HPV-infected epithelium and activation of immunocompetent cells comprised therein. Preferably, said improving the immune response as used herein induces killing of PV-infected cells by the immune system of the subject. More preferably, the term relates to inducing regression of at least one HPV-related lesion or/and at least one HPV-related cancer. It is to be understood that improving the immune response as used in accordance with the present invention may not be effective in all subjects to be treated. However, the term shall require that in a statistically significant portion of subjects the immune response is improved. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 60%, at least 70%, at least 80%, or at least 90% of the subjects of a given cohort or population.

Preferably, the aforesaid PV antigen-specific CD-8+ and/or CD4+ T-cells are PV late antigen-specific CD-8+ and/or CD4+ T-cells. More preferably, the PV antigen-specific CD-8+ and/or CD4+ T-cells are PV L1 antigen-specific CD-8+ and/or CD4+ T-cells and/or PV L2 antigen-specific CD-8+ and/or CD4+ T-cells. Even more preferably, the PV antigen-specific CD-8+ and/or CD4+ T-cells are activated PV late antigen-specific CD-8+ and/or CD4+ T-cells. Most preferably, the PV antigen-specific CD-8+ and/or CD4+ T-cells are activated PV L1 antigen-specific CD-8+ and/or CD4+ T-cells and/or PV L2 antigen-specific CD-8+ and/or CD4+ T-cells.

In a preferred embodiment, improving the immune response comprises or is increasing the number of toll like receptor (TLR) molecules expressed by cells of the subject, preferably, in case of topical application, at the site of administration of the DNA demethylating agent. Also preferably, improving the immune response comprises or is increasing the frequency of TLR-expressing cells of the subject and/or or increasing the number of cell types expressing TLR. The term "toll like receptor" or "TLR" is well-known in the art and relates to a member of the family of receptor proteins playing a key role in the innate immune system. Preferably, the TLR is TLR7 and/or TLR9, more preferably human TLR7 (Genbank Acc. NOs: AAQ88659.1 GI:37181704, NP_057646.1 GI:7706093, Q9NYK1.1 GI:20140876, AAF78035.1 GI:8575525, AAF60188.1 GI:7330281, EAW98807.1 GI:119619213, AAH33651.1 GI:21708075, and BAG55056.1 GI:194068471; mRNA Genbank Acc NO: AY358292.1 GI:37181703) and/or TLR9 (Genbank Acc. NOs: AAQ89443.1 GI:37183287, Q9NR96.2 GI:20140872, AAF78037.1 GI:8575529, BAB19259.1 GI:11761321, EAW65191.1 GI:119585595, AAH32713.1 GI:21595773, BAG55070.1 GI:194068499, ABW37078.1 GI:158346923, ABW37077.1 GI:158346921, ABW37076.1 GI:158346919, ABW37075.1 GI:158346917, ABW37074.1 GI:158346915, and Q9EQU3.3 GI:363548499, mRNA Genbank Acc NO: AY359085.1 GI:37183286). Preferably, the cells in which the number of TLR molecules is increased and/or in which the frequency of TLR-expressing cells increases are fibroblasts, epithelial cells, monocytes, macrophages, dendritic cells, and/or B lymphocytes.

The term "killing" PV-infected cells, as used herein, relates to inducing termination of viability of a cell. Preferably, killing a cell is inducing lysis, necrosis, or, more preferably, apoptosis of said cell. More preferably, killing is inducing apoptosis by cytotoxic T-cells.

The DNA demethylating agent according to the present invention is administered to a subject or in vitro to a population of host cells of the present invention. Preferably, the DNA demethylating agent is administered systemically to a subject. More preferably, the DNA demethylating agent is administered topically. Most preferably, the DNA demethylating agent is administered topically onto a body surface comprising at least a part of an HPV related lesion. It is understood by the skilled person that in case the DNA demethylating agent according to the present invention is administered in vitro to a population of host cells, said host cells may then be re-introduced into the subject in order to achieve improving the immune response to PV. Alternatively, said host cells may be used for in vitro stimulation of white blood cells obtained from said subject, which may then be re-introduced into the subject in order to achieve improving the immune response. It is also understood by the skilled person that host cells to which the DNA demethylating agent was administered and/or stimulated white blood cells may also be administered to a subject different from the subject the host cells and/or white blood cells were derived from (allogeneic administration). However administration to the subject of the host cells and/or white blood cells were derived from (autologous administration) is preferred.

Advantageously, it was found in the work underlying the present invention that demethylating agents also allow to activate the expression of the late viral genes L1 and L2 in the basal cells of the epithelium. This in turn results in proficient presentation of the respective protein products to antigen presenting cells, triggers a profound T-cell response and finally may allow for spontaneous regression of the HPV-infection and the respective epithelial lesion. Surprisingly, and in contrast to the expectations in the prior art, expression of PV oncogenes E6 and E7 was found not to increase, such that treatment with a DNA demethylating agent can be considered to not increase the risk of malignant transformation of PV-related lesions.

The definitions made above apply mutatis mutandis to the following. Additional definitions and explanations made further below also apply for all embodiments described in this specification mutatis mutandis.

The present invention also relates to a pharmaceutical composition for use according to claim 11; further disclosure relates to a pharmaceutical composition comprising a DNA demethylating agent and at least a pharmaceutical carrier for use in increasing the immune response to human papillomavirus (PV) of a subject infected with PV.

The term "pharmaceutical composition" as used herein comprises the compound or compounds of the present invention and one or more pharmaceutically acceptable carrier. The compounds of the present invention can be formulated as pharmaceutically acceptable salts. Acceptable salts comprise acetate, methylester, HCl, sulfate, chloride and the like. The pharmaceutical compositions are, preferably, administered topically or systemically. Suitable routes of administration conventionally used for drug administration are oral, intravenous, or parenteral administration as well as inhalation. However, depending on the nature and mode of action of a compound, the pharmaceutical compositions may be administered by other routes as well.

Moreover, the compounds can be administered in combination with other drugs either in a common pharmaceutical composition or as separated pharmaceutical compositions wherein said separated pharmaceutical compositions may be provided in form of a kit of parts. Preferably, the present invention also contemplates a pharmaceutical composition comprising more than one DNA demethylating agent. Preferably, the pharmaceutical composition comprises at least one covalent DNMT inhibitor and at least one non-covalent DNMT inhibitor. Also preferably, the present invention contemplates a pharmaceutical composition comprising at least one DNA demethylating agent and at least one additional enhancer of the immune response, e.g. imiquimod (3-(2-methylpropyl)-3,5,8-triazatricyclo[7.4.0.02,6]trideca-1(9),2(6),4,7,10,12-hexaen-7-amine).

The compounds are, preferably, administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

The carrier(s) must be acceptable in the sense of being compatible with the other ingredients of the formulation and being not deleterious to the recipient thereof. The pharmaceutical carrier employed may be, for example, either a solid, a gel or a liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania. Preferably, the pharmaceutically acceptable carrier comprises at least one component selected from stearic acid, isopropyl palmitate, propylene glycol, potassium sorbate, sorbic acid, triethanolamine lauryl sulfate,dimethyl-sulfoxide, cyclodextrin, ethylenediaminetetraacetic acid (EDTA), hydroxyethyl cellulose, methylparaben, niacinamide, phenoxyethanol, propylparaben and purified water. Particularly in topical application, the carrier, preferably, is or comprises an organic solvent, more preferably is or comprises dimethyl-sulfoxide (DMSO). Also preferably, the carrier is or comprises nanoparticles.

The diluent(s) is/are selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like.

A therapeutically effective dose refers to an amount of the compounds to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. A typical dose for systemic application can be, for example, in the range of 1 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. However, depending on the subject and the mode of administration, the quantity of substance administration may vary over a wide range to provide from about 0.01 mg per kg body mass to about 10 mg per kg body mass. For topical application, it is envisaged that the DNA demethylating agent is present in a concentration of 0,001 to 50% (w/w) in the pharmaceutical composition. Also preferably, in topical application, the pharmaceutical composition is provided on a polymer carrier. Preferably, the polymer carrier comprises at least one polymer selected from the list consisting of cellulose, polyethylene, polypropylene, poly(dimethylsiloxane), ethylene vinyl acetate copolymer, polycarbophil, hydroxypropyl methylcellulose and poly(acrylic acid). More preferably, the polymer carrier essentially consists of a polymer selected from the list consisting of cellulose, polyethylene, polypropylene, poly(dimethylsiloxane), ethylene vinyl acetate copolymer, polycarbophil, hydroxypropyl methylcellulose and poly(acrylic acid). As used herein, the term "essentially consists of" relates to the polymer carrier comprising, apart from the specific polymer, only minor impurities, impurities being substances different from the specific polymer. Preferably, the polymer carrier comprises at most 20% impurities; more preferably, the polymer comprises at most 10% impurities; most preferably, the polymer carrier comprises at most 5% impurities.

In a preferred embodiment, the pharmaceutical composition further comprises an antigen inducing an immune response to a PV L1 polypeptide and/or to a PV L2 polypeptide. More preferably, said antigen comprises a PV L1 polypeptide or an immunogenic epitope thereof and/or a PV L2 polypeptide or an immunogenic epitope thereof. Still more preferably, said antigen is a virus-like particle comprising a PV L1 polypeptide or an immunogenic epitope thereof and/or a PV L2 polypeptide or an immunogenic epitope thereof. Most preferably, said antigen is a PV-like particle comprising a PV L1 polypeptide or an immunogenic epitope thereof and/or a PV L2 polypeptide or an immunogenic epitope thereof.

The pharmaceutical compositions and formulations referred to herein are administered at least once in order to achieve the effect recited in this specification. However, the said pharmaceutical compositions may be administered more than one time, for example from one to four times daily up to a non-limited number of days.

Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adapted to the mode of administration, i.e. in the forms of tablets, capsules, suppositories, solutions, suspensions or the like. Dosage recommendations shall be indicated in the prescribers or users instructions in order to anticipate dose adjustments depending on the considered recipient.

The present invention further relates to an in vitro use of a DNA demethylating agent according to claim 13; further disclosure relates to a use of a DNA demethylating agent for increasing the immune response to PV of a subject infected with PV.

Further, the present disclosure relates to a method for increasing the immune response to a PV of a subject infected with said PV, comprising
a) contacting PV-infected cells of said subject infected with PV with a DNA demethylating agent, and
b) thereby, increasing the immune response to PV of said subject infected with PV.

The method for increasing the immune response to PV, preferably, is an in vivo method. However, one or more steps of the method for increasing the immune response to PV may also be performed in vitro. E.g. the step of contacting PV-infected cells of said subject infected with PV with a DNA demethylating agent may also be performed in vitro on cells obtained from said subject. As the skilled person will understand, in such a case the additional step of readministration of the cells such treated to a subject is required in order to increase the immune response of said subject. As detailed herein above, PV-infected cells contacted with a DNA demethylating agent in vitro may also be used to stimulate white blood cells in vitro according to standard methods. The skilled person understands that in this case the readministration of said PV-infected cells contacted with a DNA demethylating agent and/or said white blood cells stimulated therewith is required to increase the immune response of a subject. Moreover, the method for increasing the immune response to PV may comprise steps in addition to those explicitly mentioned above. For example, further steps may relate, e.g., to administering the DNA demethylating agent to a subject for step a), or providing additional immunostimulatory treatment before or in step b). Moreover, one or more of said steps may be performed or assisted by automated equipment.

The term "contacting" as used in the context of the methods of the present invention is understood by the skilled person. Preferably, the term relates to bringing a compound or composition of the present invention in physical contact with a subject or cell and thereby, e.g. allowing a PV-infected cell and the compound or composition to interact. As the skilled person will understand, in as far as the term "contacting" relates to contacting a cell of a subject with a DNA demethylating agent of the present invention, contacting may be administering a DNA demethylating agent to said subject as specified herein above. Thus, preferably, contacting PV-infected cells of said subject infected with PV with a DNA demethylating agent may comprise systemic or topical application of said DNA demethylating agent as specified above.

In a disclosure, the method for increasing the immune response to a PV further comprises contacting said subject with an antigen inducing an immune response to a PV L1 polypeptide and/or to a PV L2 polypeptide.

The present invention further relates to an in vitro method of killing a PV-infected cell according to claim 14; further dsiclosure relates to a method of killing a PV-infected cell, comprising
a) contacting said PV-infected cell with a DNA demethylating agent,
b) contacting said PV-infected cell with at least one PV antigen specific T-cell, and
c) thereby, killing said PV-infected cell.

The method of killing a PV-infected cell is an in vitro method. Moreover, the method of killing a PV-infected cell may comprise steps in addition to those explicitly mentioned above. For example, further steps may relate, e.g., to identifying PV-infected cells and/or at least one PV related lesion or/and at least one PV-positive cancer in a subject for step a), or administering the DNA demethylating agent to said subject in step b). Moreover, one or more of said steps may be performed or assisted by automated equipment.

Preferably, the PV-infected cell is a PV-positive tumor cell, e.g., preferably, comprised in a PV related lesion. More preferably, said PV-infected cell is a PV-positive cancer cell, e.g., preferably, comprised in a PV-related cancer. More preferably, the PV-infected cell is a PV-positive cervix carcinoma cell.

Moreover, the present disclosure relates to a method for preventing or/and treating PV-positive cancer in a subject afflicted with at least one PV-related lesion or/and at least one PV-related cancer, comprising
a) contacting at least a part of said at least one PV related lesion with a DNA demethylating agent, and
b) thereby, preventing PV-positive cancer in a subject afflicted with at least one PV-related lesion.

The method for preventing or/and treating PV-positive cancer may comprise steps in addition to those explicitly mentioned above. For example, further steps may relate, e.g., to identifying and/or locating a PV-related lesion or cancer for step a), or surgical removal of any residual tumor before step b). Moreover, one or more of said steps may be performed by automated equipment.

The tem "preventing" refers to retaining health with respect to the diseases or disorders referred to herein for a certain period of time in a subject. It will be understood that the said period of time may depend on the amount of the drug compound which has been administered and on individual factors of the subject discussed elsewhere in this specification. It is to be understood that prevention may not be effective in all subjects treated with the compound according to the present invention. However, the term requires that a statistically significant portion of subjects of a cohort or population are effectively prevented from suffering from a PV related cancer. Preferably, a cohort or population of subjects is envisaged in this context which normally, i.e. without preventive measures according to the present invention, would develop PV related cancer as referred to herein. Whether a portion is statistically significant can be determined without further ado by the person skilled in the art using various well known statistic evaluation tools discussed elsewhere in this specification.

The term "treating" refers to ameliorating the diseases or disorders referred to herein or the symptoms accompanied therewith to a significant extent. Said treating as used herein also includes an entire restoration of the health with respect to the diseases or disorders referred to herein. It is to be understood that treating as used in accordance with the present invention may not be effective in all subjects to be treated. However, the term shall require that a statistically significant portion of subjects suffering from a disease or disorder referred to herein can be successfully treated. Whether a portion is statistically significant can be determined without further ado by the person skilled in the art using various well known statistic evaluation tools as described herein above.

In a disclosure the method for preventing or/and treating PV-positive cancer further comprises contacting said subject with an antigen inducing an immune response to a PV L1 polypeptide and/or to a PV L2 polypeptide as specified herein above.

In a further disclosure, the method for preventing or/and treating PV-positive cancer is a method of treating PV-related oropharyngeal carcinoma and comprises systemic administration of a DNA demethylating agent to a subject. In a further disclosure, said method of treating PV-related oropharyngeal carcinoma comprises systemic administration of a DNA demethylating agent and contacting said subject with an antigen inducing an immune response to a PV L1 polypeptide and/or to a PV L2 polypeptide to said subject.

In a further disclosure, the method for preventing or/and treating PV-positive cancer is a method for treating a PV-related lesion or of a PV-related cancer in the anogenital tract of a subject and said method comprises topical administration of a DNA demethylating agent. In a further disclosure, said method for treating a PV-related lesion or of a PV-related cancer in the anogenital tract of a subject comprises topical administration of a DNA demethylating agent and contacting said subject with an antigen inducing an immune response to a PV L1 polypeptide and/or to a PV L2 polypeptide to said subject.

Further, the present disclosure relates to a use of a DNA demethylating agent for the manufacture of a medicament for increasing the immune response to PV of a subject infected with PV, for killing PV-positive tumor cells, or for preventing PV-positive cancer in a subject afflicted with at least one PV-related lesion.

Moreover, the present disclosure relates to a DNA demethylating agent for use in preventing malignant transformation of a PV-related lesion.

The term "malignant transformation" is known to the skilled person and relates to the process of a PV-related lesion developing into a PV-related cancer.

### Figure Legends

Fig. 1: Schematic drawing of the HPV life cycle in replicating and transforming HPV infections. C: columnar epithelium, S: squamous epithelium, L: latent infection, P: permissive infection, T: transforming infection.

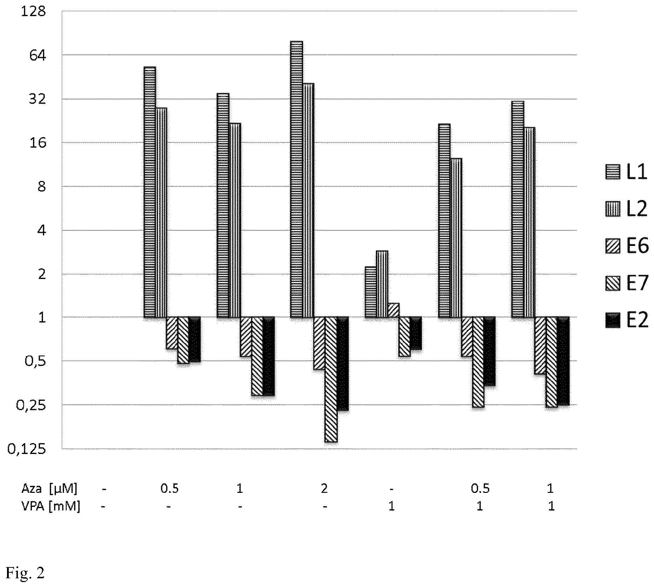

Fig. 2: Effect of DNA demethylating agents on HPV gene expression in Caski cells qRT-PCR analysis of HPV L1, L2, E6, E7 and E2 gene expression in Caski cells treated with different concentrations of Aza and VPA. Y-Axis: relative gene expression as compared to untreated control (arbitrary units), Aza: 5-aza-2'-deoxycytidine, VPA: valproic acid.

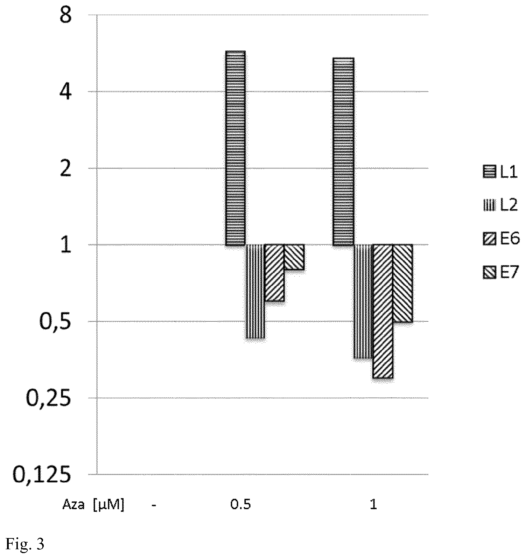

Fig. 3: Effect of DNA demethylating agents on HPV gene expression in SiHa cells qRT-PCR analysis of HPV L1, L2, E6, E7 and E2 gene expression in Caski cells treated with different concentrations of Aza. Y-Axis: relative gene expression as compared to untreated control (arbitrary units), Aza: 5-aza-2'-deoxycytidine.

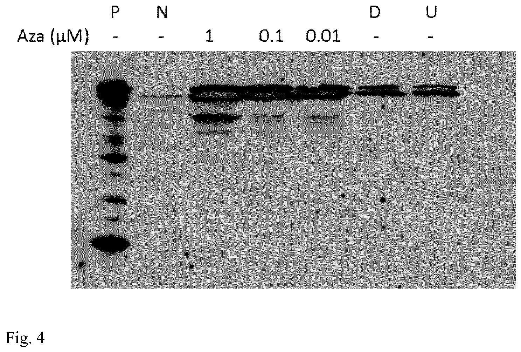

Fig. 4: Detection of HPV L1 capsid protein by immunoblotting
   Western blot analysis of L1 expression in Caski cells treated for 28 days with 5-aza-2'-deoxycytidine. Significant increase of L1 protein expression level is noted upon 5-aza-2'-deoxycytidine treatment. The band at approximately 55 kDa corresponds to L1 protein detected by anti HPV16 L1 polyclonal rabbit antibodies. P: positive control (L1-VLP, recombinant L1 protein), N: negative control (C33A cells, a HPV-16-negative cell line), D: DMSO (carrier) control, U: untreated control.

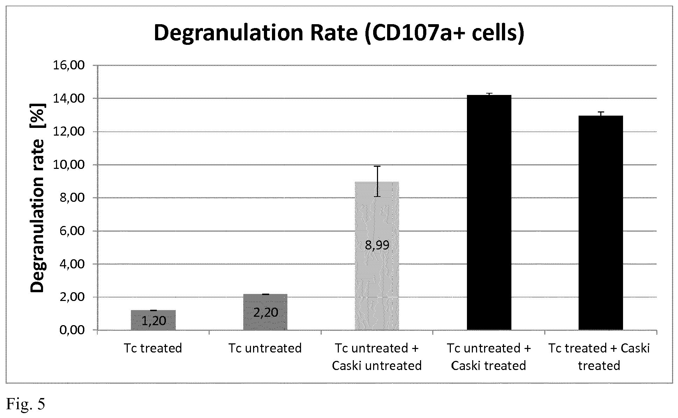

Fig. 5: Enhanced cytolytic activity of T-cells stimulated in vitro with L1-derived peptides towards Caski cells treated with azacitidine and thus expressing the L1-antigen. Shown is the degranulation rate exhibited by various cells or combinations of cells, expressed as the fraction of CD107a/CD8 double positive cells compared to the number of CD8 positive cells.

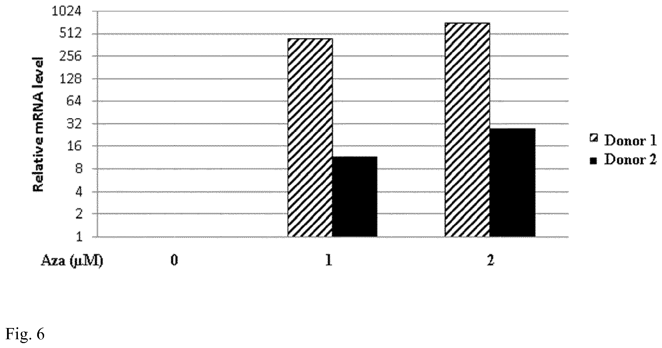

Fig. 6: Relative induction of TLR 7 expression in PBLs by 5-aza-2'-deoxycytidine treatment. qRT-PCR analysis of TLR 7 gene expression in PBL cells treated with different concentrations of 5-aza-2'-deoxycytidine (DAC). Y-Axis: relative gene expression as compared to DMSO (solvent) control (arbitrary units).

The following Examples shall merely illustrate the invention. They shall not be construed, whatsoever, to limit the scope of the invention.

### Example 1: Effect of 5-aza-2'-deoxycytidine on HPV gene expression

### Cell culture

Caski and SiHa cells were cultured in Dulbecco's modified Eagle's medium (DMEM) medium (Invitrogen Life Technologies), which was supplemented with 10% heat inactivated fetal bovine serum (FBS) (Gibco^{®}, Invitrogen Life Technologies), 100 µg/ml streptomycin, 100 U/ml penicillin (Invitrogen Life Technologies) and cultured at 37°C in a humidified 5% CO2 incubator. Cells were detached by trypsinization (×1 trypsin EDTA, Invitrogen Life Technologies).

Cells were seeded to 50% confluency and cultivated in the absence and presence of 5-aza-2'-deoxycytidine (5-Aza-CdR, Sigma) and valproic acid (VPA, Sigma) at concentrations of 0,5. 1 and 2 µM for 5-Aza-CdR and 1mM and 2 mM for VPA for three days. Media was changed daily with fresh drug application.

### Preparation of total RNA

Total RNA was extracted from Caski cells using RNeasy kit (Qiagen) according to the manufacturer's instructions, with the addition of an extra DNase I treatment step. Total RNA of 1 µg for each sample was converted to cDNA for quantitative real-time PCR (qPCR) using SuperScript II and 1:1 mixture of oligo (dT) and random hexamers primers (Invitrogen Life Technologies).

### Quantification of RNA

Quantitative Real-time RT-PCR was performed with primers to detect human papillomavirus 16 (HPV16) genes expression (NCBI accession number is AF125673.1). Primers used in qPCR are listed in the Table 1.

**Table 1: Primers used in qPCR**

| **Name** | **Sequence (5'-3')** | **SEQ ID NO:** |
|---|---|---|
| **L1 Forward** | GGTGTTGAGGTAGGTCGTGG | 1 |
| **L1 Reverse** | CACACCTGCATTTGCTGCAT | 2 |
| **L2 Forward** | AGGAATTGGAACAGGGTCGG | 3 |
| **L2 Reverse** | AGGGGGTCTTACAGGAGCAA | 4 |
| **E2 Forward** | CAGACGACTATCCAGCGACC | 5 |
| **E2 Reverse** | GCAGTGAGGATTGGAGCACT | 6 |
| **E6 Forward** | ACTGCGACGTGAGGTGTATTAAC | 7 |
| **E6 Reverse** | TGGAATCTTTGCTTTTTGTCC | 8 |
| **E7 Forward** | CAGCTCAGAGGAGGAGGATG | 9 |
| **E7 Reverse** | GCCCATTAACAGGTCTTCCA | 10 |
| **β-actin Forward** | ATGTGGCCGAGGACTTTGATT | 11 |
| **β-actin Reverse** | AGTGGGGTGGCTTTTAGGATG | 12 |
| **TLR7 Forward** | AAGCCCTTTCAGAAGTCCAAGTT | 16 |
| **TLR7 Reverse** | GGTGAGCTTGCGGGTTTGT | 17 |

The human β-actin gene (NCBI Acc No: NM_001101.3 GI:168480144) expression was used as a normalization control.

Quantitative PCR was performed in triplicates in a 96-well plate format on an ABI StepOnePlus^{™} Real-Time PCR Systems (Applied Biosystems) using Power SYBR Green Master Mix (Applied Biosystems), forward and reverse primers (Thermo Scientific) at 150 nM final concentration, and cDNA template or water for the no-template controls. Cycling conditions were 95 °C for 20 sec, followed by 40 cycles of 95 °C for 3 sec and 60 °C for 30 sec on the StepOnePlus^{™} Real-Time PCR System (Applied Biosystems). Threshold cycle PCR values (Ct), were obtained during exponential amplification. Threshold cycle differences between duplicate reactions smaller than 0.5 cycles were considered acceptable. The HPV16 gene expression, in relative units obtained by comparing Ct value of target gene with normalization control gene.

### Example 2: Effect of 5-aza-2'-deoxycytidine treatment on HPV L1 protein production

5-aza-2'-deoxycytidine was added daily to cultures of Caski cells at concentrations 0.01, 0.1 and 1µM. Caski cells were cultured with medium changed every 24 hours. Treatments were stopped at day 28 and the cells were grown in the absence of drugs for 14 days. The same culture conditions were used for untreated and DMSO treated Caski cells. Samples were collected on days 7, 14, 21, 28 and 38 for genomic DNA and total RNA purifications.

Protein extracts were also obtained from Caski cells on days 7, 14, 21, 28 and 38 of treatment. Cells were lysed in RIPA buffer containing Protease Inhibitor Cocktail (Sigma). Whole-cell lysates were prepared in denaturing SDS sample buffer and subjected to 12% SDS-PAGE. Proteins were transferred to PVDF Membrane (Invitrogen) and the blots were blocked with 5% non-fat dry milk in TBS-T buffer. The anti HPV16 L1 polyclonal rabbit antibodies (kindly provided by Dr Martin Müller, DKFZ, Germany) were used to detect the HPV16 L1 protein. Horseradish peroxidase conjugated secondary antibodies (1:2000 dilution, Anti-rabbit IgG, HRP-linked Antibody #7074 (Cell Signaling) were detected using Western Blotting Chemiluminescence Luminol Reagent (Santa Cruz).

### Example 3: Degranulation Assay

### Generation of dendritic cells deriving from monocytes

PBMCs were obtained from blood of a healthy, HLA-A^{∗}0201 positive donor by density gradient centrifugation with lymphocyte separation medium (PAA, Pasching, Austria). PBMCs were resuspended in RPMI medium (Gibco, Paisley, UK), containing 5% human serum and seeded in cell culture flasks. After 4 hours of adherence at 37°C the non-adherent cells were removed. To stimulate the differentiation of monocytes into dendritic cells, the adherent fraction of cells was cultured in CellGro Medium (CellGenix, Freiburg, Germany) containing GM-CSF (1000 U/ml, Promokine, Heidelberg, Germany) and IL-4 (666,7 U/ml, Promokine, Heidelberg, Germany) for 5-7 days. Then, IL-4 and GM-CSF were added again together with a terminal differentiation-inducing cocktail containing the proinflammatory cytokines IL-6 (1000 U/ml Promokine, Heidelberg, Germany), IL-1β (10 ng/ml Promokine, Heidelberg, Germany), PGE-2 (lmg/ml, Promokine, Heidelberg, Germany) and TNF-α (10 ng/ml, Promokine, Heidelberg, Germany) were added and cells were incubated overnight. The next day, dendritic cells were harvested and used for T cell stimulation.

### Isolation of T-cells from PBMCs

T cells were obtained from the same donor by using the non-adherent fraction of PBMCs following the 4 hours adherence in cell culture flasks. Pan T cell isolation was performed based on MACS Pan T cell isolation Kit II following the manufacturer's protocol (Miltenyi Biotec, Bergisch Gladbach, Germany).

L1-Peptides used for T cell stimulation:

| | | |
|---|---|---|
| HPV 16 L1 (aa 12-22): | YLPPVPVSKV | SEQ ID NO:13 |
| HPV 16 L1 (aa 97-106): | RLVWACVGV | SEQ ID NO:14 |
| HPV 16 L1 (aa 323-332): | ICWGNQLFV | SEQ ID NO:15 |

These synthetic peptides were synthesized by Genaxxon, Ulm, Germany.

### Stimulation of T-cells with dendritic cells

For the stimulation of T cells with L1-peptides, dendritic cells and T cells were used in a ratio of 1:10. For peptide-loading, dendritic cells were incubated in CellGro medium in presence of pooled Ll-peptides (20µg/ml each) (HLA-A^{∗}0201-restricted) and Lipofectamine 2000 (20µg/ml, Invitrogen, Paisley, UK) for 2-3 hours at 37°C, 5% CO2. Dendritic cells were then irradiated with 30 Gy, washed twice and then cultured together with T cells in medium supplemented with Insulin-Transferrin-Selenium (lx, Gibco, Paisley, UK) and IL-2 and IL-7 (20 U/ml each, Promokine, Heidelberg, Germany). Stimulation of T cells with peptide-loaded dendritic cells was repeated weekly using freshly isolated PBMCs for dendritic cell generation in order to obtain a number of four stimulations in total over a period of 24 days.

### CD107a Mobilization Assay

To monitor the degranulation of L1-stimulated T cells (effectors) induced by Caski cells (targets), Caski cells pretreated with different concentrations of 5-Aza-2'-deoxycytidine (0.1 µM and 1.0 µM) for 12 days or untreated cells were used. Effectors and targets were used at a ratio of 1:1 and were cultured for 5 hours at 37°C, 5% CO2 in presence of a PE-labeled CD107a antibody (BD Pharmingen, Heidelberg, Germany). After one hour, 5 µg/ml Brefeldin A was added. For CD107a/CD8 double staining, cells were harvested and stained with a FITC-labeled CD8 (BD Pharmingen, Heidelberg, Germany) antibody. Degranulation rate was measured as the fraction of CD107a/CD8 double staining cells relative to total CD8 positive cells (Fig. 5). It was found that 5-Aza-2'-deoxycytidine-treatment of Caski cells increased the fraction of T cells undergoing degranulation.

### Example 4: PBMC stimulation with 5-Aza-2'-deoxycytidine.

PBMCs were obtained from blood of two healthy donors by density gradient centrifugation with lymphocyte separation medium (PAA, Pasching, Austria) and washed with RPMI medium (Gibco, Paisley, UK). For stimulation of PBMCs with 5-Aza-2'-deoxycytidine cells were resuspended in a IMDM-based medium (Gibco, Paisley, UK) optimized for lymphocytes containing 1x Insulin-Transferrin-Selenium (Gibco, Paisley, UK) and Interleukins 4, 2 and 7 ((20 U/ml each, , Promokine, Heidelberg, Germany). 2×10⁶ cells per well of a 24-well plate were seeded in 2 ml of the complete medium. 5-Aza-2'-deoxycytidine was added and tested in different concentrations. PBMCs untreated and treated with DMSO served as negative controls.
- 5-Aza-2'-deoxycytidine: 1µM
- 5-Aza-2'-deoxycytidine: 2 µM
- DMSO control: 0,4 µl
- negative control (untreated)

PBMCs were cultured for 72 hours. Substances were added every 24 hours at the same concentrations as described above and without changing the medium (in total 3 doses). After 72 hours, total RNA was extracted an analyzed for TLR-7 expression as described in Example 1.

## Claims

1. A DNA demethylating agent for use in improving the immune response to a papillomavirus (PV) of a subject infected with said PV, wherein said improving the immune response is increasing the frequency of PV late antigen-specific CD-8+ and/or CD4+ T-cells in the blood of said subject.

2. The DNA demethylating agent for use of claim 1, wherein said DNA demethylating agent is a chemical compound inhibiting the activity of human DNA methyltransferase (DNMT) *in vitro* by at least 50% when present at a concentration of 1 µM in the assay mixture, or wherein said DNA demethylating agent is a chemical compound causing the measurable specific activity of human DNA methyltransferase (DNMT) in cell extracts from MCF-7 cells to decrease by at least 10% when present in the culture medium at a concentration of 1 µM for 5 days.

3. The DNA demethylating agent for use of claim 1 or 2, wherein said DNA demethylating agent is selected from the list consisting of 5-Aza-2'-deoxycytidine; 5-Azacytidine; Arabinosyl-5-azacytidine; 5-6-Dihydro-5-azacytidine; 5-Fluoro-2'-deoxycytidine; Epigallocatechin-3- gallate; 1-hydrazinylphthalazine (Hydralazine), 4-amino-N-(2-diethylaminoethyl) benzamide (Procainamide); 2-(diethylamino)ethyl 4-aminobenzoate (Procaine); 2-(1H)-pyrimidinone riboside (Zebularine); 2-amino-4-([[(2S,3S,4R,5R)-5-(6-amino-2-R¹-9H-purin-9-yl)-3,4-dihydroxytetrahydrofuran-2-yl]methyl]sulfanyl)butanoic acid with R¹ being chloro-, fluoro-, iodo-, methoxy-, methyl- or methylsulfanyl-; S-Tubercidinylhomocysteine; S-(N-(2-biphenyl-4-ylethyl)adenosyl)-L-homocysteine; S-(N-(2-biphenyl-4-ylethyl)-2-chloroadenosyl)-L-homocysteine; S-(N-phenylpropyladenosyl)-L-homocysteine; 8-aza-S-adenosyl-L-homocysteine; S-(N-(3,5-dimethoxybenzyl)adenosyl)-L-homocysteine; S-(N-(pyridin-4-ylmethyl)adenosyl)-L-homocysteine; S-(N-phenylethyladenosyl)-L-homocysteine; S-nebularinehomocysteine; S-(N-(2-biphenyl-4-ylethyl)-2-chloroadenosyl)-L-homocysteine; 1-deaza-S-adenosyl-L-homocysteine; 3-deaza-S-adenosyl-L-homocysteine; S-(1-deazaadenosyl)-L-homocysteine; S-(N-benzyladenosyl)-L-homocysteine; S-(N-phenyladenosyl)-L-homocysteine; and 1,2-dihydropyrimidin-2-one-5-methylene-(methylsolfonium)-adenosyl.

4. The DNA demethylating agent for use of any one of claims 1 to 3, wherein said DNA demethylating agent is selected from the list consisting of 5-Aza-2'-deoxycytidine, 5-Azacytidine, and Arabinosyl-5-azacytidine.

5. The DNA demethylating agent for use of any one of claims 1 to 4, wherein said subject infected with PV is afflicted with at least one PV related lesion or/and with at least one PV-positive cancer.

6. The DNA demethylating agent for use of claim 5, wherein said PV related lesion is a PV-positive lesion selected from the list consisting of warts, exophytic growing papillomas, condylomata, inverted papillomas, pre-neoplastic or neoplastic PV-induced lesions, cervical neoplasias, skin neoplasias, neoplasias of the anal epithelium, neoplasias of the head and neck region, preferably of the oropharynx, or neoplasias of the tonsils.

7. The DNA demethylating agent for use of any one of claims 1 to 6, wherein said DNA demethylating agent is for topical application.

8. The DNA demethylating agent for use of any one of claims 1 to 7, wherein said PV antigen-specific T-cells are PV late antigen-specific CD-8+ T-cells.

9. The DNA demethylating agent for use of any one of claims 1 to 8, wherein said improving the immune response is increasing the frequency of activated PV antigen-specific CD-8+ T-cells in the blood of said subject.

10. The DNA demethylating agent for use of claim 9, wherein said activated PV antigen-specific CD-8+ T-cells are activated PV L1 antigen-specific CD-8+ T-cells and/or PV L2 antigen-specific CD-8+ T-cells.

11. A pharmaceutical composition comprising a DNA demethylating agent and at least a pharmaceutical carrier for use in increasing the immune response to papillomavirus (PV) of a subject infected with PV, wherein said improving the immune response is increasing the frequency of PV late antigen-specific CD-8+ and/or CD4+ T-cells in the blood of said subject.

12. The pharmaceutical composition for use of claim 11, wherein the DNA demethylating agent is present in a concentration of 0,001 to 50%.

13. In vitro use of a DNA demethylating agent for increasing the frequency of PV late antigen-specific CD-8+ and/or CD4+ T-cells in a blood sample.

14. An in vitro method of killing a PV-infected cell, comprising
contacting said PV-infected cell with a DNA demethylating agent,
contacting said PV-infected cell with PV late antigen specific CD8+ and/or CD4+ T-cells, and
thereby, killing said PV-infected cell.

## Patentansprüche

1. DNA-Demethylierungsmittel zur Verwendung beim Verbessern der Immunantwort eines mit einem Papillomavirus (PV) infizierten Individuums gegen das PV, wobei durch das Verbessern der Immunantwort die Häufigkeit von für spätes PV-Antigen spezifischen CD-8+- und/oder CD4+-T-Zellen im Blut des Individuums erhöht wird.

2. DNA-Demethylierungsmittel zur Verwendung nach Anspruch 1, wobei es sich bei dem DNA-Demethylierungsmittel um eine chemische Verbindung handelt, die die Aktivität menschlicher DNA-Methyltransferase (DNMT) *in vitro* um wenigstens 50% hemmt, wenn es im Testgemisch in einer Konzentration von 1 µM vorliegt, oder wobei es sich bei dem DNA-Demethylierungsmittel um eine chemische Verbindung handelt, die dazu führt, dass die messbare spezifische Aktivität menschlicher DNA-Methyltransferase (DNMT) in Zellextrakten aus MCF-7-Zellen um wenigstens 10% abnimmt, wenn es im Kulturmedium 5 Tage in einer Konzentration von 1 µM vorliegt.

3. DNA-Demethylierungsmittel zur Verwendung nach Anspruch 1 oder 2, wobei das DNA-Demethylierungsmittel ausgewählt ist aus der Liste bestehend aus 5-Aza-2'-desoxycytidin; 5-Azacytidin; Arabinosyl-5-azacytidin; 5-6-Dihydro-5-azacytidin; 5-Fluor-2'-desoxycytidin; Epigallocatechin-3-gallat; 1-Hydrazinylphthalazin
(Hydralazin), 4-Amino-N-(2-diethylaminoethyl)-benzamid (Procainamid); 4-Aminobenzoesäure-2-(*N*,*N-*diethylamino)ethylester (Procain); 2-(1H)-Pyrimidinon-ribosid (Zebularin); 2-Amino-4-([[(2S,3S,4R,5R)-5-(6-amino-2-R¹-9H-purin-9-yl)-3,4-dihydroxytetrahydrofuran-2-yl]methyl]sulfanyl)butansäure, wobei R¹ für Chlor-, Fluor-, Iod-, Methoxy-, Methyl- oder Methylsulfanylsteht; S-Tubercidinylhomocystein; S-(N-(2-Biphenyl-4-ylethyl)adenosyl)-L-homocystein; S-(N-(2-Biphenyl-4-yl-ethyl)-2-chloradenosyl)-L-homocystein; S-(N-Phenyl-propyladenosyl)-L-homocystein; 8-Aza-S-adenosyl-L-homocystein; S-(N-(3,5-Dimethoxybenzyl)adenosyl)-L-homocystein; S-(N-(Pyridin-4-ylmethyl)adenosyl)-L-homocystein; S-(N-Phenylethyladenosyl)-L-homocystein; S-Nebularinhomocystein; S-(N-(2-Biphenyl-4-ylethyl)-2-chloradenosyl)-L-homocystein; 1-Deaza-S-adenosyl-L-homocystein; 3-Deaza-S-adenosyl-L-homocystein; S-(1-Deazaadenosyl)-L-homocystein; S-(N-Benzyladenosyl)-L-homocystein; S-(N-Phenyladenosyl)-L-homocystein; und 1,2-Dihydropyrimidin-2-on-5-methylen(methylsolfonium)-adenosyl.

4. DNA-Demethylierungsmittel zur Verwendung nach einem der Ansprüche 1 bis 3, wobei das DNA-Demethylierungsmittel ausgewählt ist aus der Liste bestehend aus 5-Aza-2'-desoxycytidin, 5-Azacytidin und Arabinosyl-5-azacytidin.

5. DNA-Demethylierungsmittel zur Verwendung nach einem der Ansprüche 1 bis 4, wobei das mit PV infizierte Individuum von wenigstens einer Läsion in Verbindung mit PV oder/und von wenigstens einer PV-positiven Krebserkrankung betroffen ist.

6. DNA-Demethylierungsmittel zur Verwendung nach Anspruch 5, wobei es sich bei der Läsion in Verbindung mit PV um eine PV-positive Läsion ausgewählt aus der Liste bestehend aus Warzen, exophytisch wachsenden Papillomen, Kondylomen, inversen Papillomen, präneoplastischen oder neoplastischen PV-induzierten Läsionen, zervikalen Neoplasien, Hautneoplasien, Neoplasien des Analepithels, Neoplasien im Kopf- und Halsbereich, bevorzugt in Mund und Rachen, oder Neoplasien der Rachenmandeln handelt.

7. DNA-Demethylierungsmittel zur Verwendung nach einem der Ansprüche 1 bis 6, wobei das DNA-Demethylierungsmittel zur topischen Verabreichung vorgesehen ist.

8. DNA-Demethylierungsmittel zur Verwendung nach einem der Ansprüche 1 bis 7, wobei es sich bei den für PV-Antigen spezifischen T-Zellen um für spätes PV-Antigen spezifische CD-8+-T-Zellen handelt.

9. DNA-Demethylierungsmittel zur Verwendung nach einem der Ansprüche 1 bis 8, wobei das Verbessern der Immunantwort im Erhöhen der Häufigkeit aktivierter für PV-Antigen spezifischer CD-8+-T-Zellen im Blut des Individuums besteht.

10. DNA-Demethylierungsmittel zur Verwendung nach Anspruch 9, wobei es sich bei den aktivierten für PV-Antigen spezifischen CD-8+-T-Zellen um aktivierte für PV-L1-Antigen spezifische CD-8+-T-Zellen und/oder für PV-L2-Antigen spezifische CD-8+-T-Zellen handelt.

11. Pharmazeutische Zusammensetzung, umfassend ein DNA-Demethylierungsmittel und wenigstens einen pharmazeutischen Träger zur Verwendung beim Steigern der Immunantwort eines mit Papillomavirus (PV) infizierten Individuums gegen PV, wobei durch das Verbessern der Immunantwort die Häufigkeit von für spätes PV-Antigen spezifischen CD-8+- und/oder CD4+-T-Zellen im Blut des Individuums erhöht wird.

12. Pharmazeutische Zusammensetzung zur Verwendung nach Anspruch 11, wobei das DNA-Demethylierungsmittel in einer Konzentration von 0,001 bis 50% vorliegt.

13. In-vitro-Verwendung eines DNA-Demethylierungsmittels zum Erhöhen der Häufigkeit von für spätes PV-Antigen spezifischen CD-8+- und/oder CD4+-T-Zellen in einer Blutprobe.

14. In-vitro-Verfahren zum Abtöten einer mit PV infizierten Zelle, umfassend
In-Kontakt-Bringen der mit PV infizierten Zelle mit einem DNA-Demethylierungsmittel,
In-Kontakt-Bringen der mit PV infizierten Zelle mit für spätes PV-Antigen spezifischen CD-8+- und/oder CD4+-T-Zellen und
dadurch Abtöten der mit PV infizierten Zelle.

## Revendications

1. Agent de déméthylation d'ADN pour une utilisation dans l'amélioration de la réponse immunitaire à un papillomavirus (PV) d'un sujet infecté par ledit PV, ladite amélioration de la réponse immunitaire étant une augmentation de la fréquence des cellules T CD-8+ et/ou CD4+ spécifiques de l'antigène tardif de PV dans le sang dudit sujet.

2. Agent de déméthylation d'ADN pour une utilisation selon la revendication 1, ledit agent de déméthylation d'ADN étant un composé chimique inhibant l'activité de l'ADN méthyltransférase humaine (DNMT) *in vitro* d'au moins 50 % lorsqu'il est présent à une concentration de 1 µM dans le mélange d'analyse, ou ledit agent de déméthylation de l'ADN étant un composé chimique provoquant une diminution d'au moins 10 % de l'activité spécifique mesurable de l'ADN méthyltransférase humaine (DNMT) dans des extraits cellulaires de cellules MCF-7 lorsqu'il est présent dans le milieu de culture à une concentration de 1 µM pendant 5 jours.

3. Agent de déméthylation d'ADN pour une utilisation selon la revendication 1 ou 2, ledit agent de déméthylation d'ADN étant choisi dans la liste constituée par 5-Aza-2'-désoxycytidine ; 5-Azacytidine ; Arabinosyl-5-azacytidine ; 5-6-Dihydro-5-azacytidine ; 5-Fluoro-2'-désoxycytidine ; épigallocatéchine-3- gallate ; 1-hydrazinylphtalazine (Hydralazine), 4-amino-N-(2-diéthylaminoéthyl)benzamide (Procainamide) ; 4-aminobenzoate de 2-(diéthylamino)éthyle (Procaïne) ; 2-(1H)-pyrimidinone riboside (Zébularine) ; acide 2-amino-4-([[(2S,3S,4R,5R)-5-(6-amino-2-R¹-9H-purin-9-yl)-3,4-dihydroxytétrahydrofuran-2-yl]méthyl]sulfanyl)butanoïque, R¹ étant chloro-, fluoro-, iodo, méthoxy-, méthyl- ou méthylsulfanyl; S-Tubercidinylhomocystéine ; S-(N-(2-biphényl-4-ylethyl)adénosyl)-L-homocystéine ; S-(N-(2-biphényl-4-lyléthyl)-2-chloroadénosyl)-L-homocystéine ; S-(N-phénylpropyladénosyl)-L-homocystéine ; 8-aza-S-adénosyl-L-homocystéine ; S-(N-(3,5-diméthoxybenzyl)adénosyl)-L-homocystéine ; S-(N-(pyridin-4-ylméthyl)adénosyl)-L-homocystéine ; S-(N-phényléthyladénosyl)-L-homocystéine ; S-nébularinehomocystéine ; S-(N-(2-biphényl-4-yléthyl)-2-chloroadénosyl)-L-homocystéine ; 1-déaza-S-adénosyl-L-homocystéine ; 3-déaza-S-adénosyl-L-homocystéine ; S-(1-déazaadénosyl)-L-homocystéine ; S-(N-benzyladénosyl)-L-homocystéine ; S-(N-phényladénosyl)-L-homocystéine ; et 1,2-dihydropyrimidin-2-one-5-méthylène-(méthylsolfonium)-adénosyle.

4. Agent de déméthylation d'ADN pour une utilisation selon l'une quelconque des revendications 1 à 3, ledit agent de déméthylation d'ADN étant choisi dans la liste constituée par 5-Aza-2'-désoxycytidine, 5-Azacytidine et Arabinosyl-5-azacytidine.

5. Agent de déméthylation d'ADN pour une utilisation selon l'une quelconque des revendications 1 à 4, ledit sujet infecté par un PV étant atteint d'au moins une lésion liée au PV et/ou d'au moins un cancer PV-positif.

6. Agent de déméthylation d'ADN pour une utilisation selon la revendication 5, ladite lésion liée au PV étant une lésion PV-positive choisie dans la liste constituée par les verrues, les papillomes exophytiques en croissance, les condylomes, les papillomes inversés, les lésions pré-néoplasiques ou néoplasiques induites par le PV, les néoplasies cervicales, les néoplasies cutanées, les néoplasies de l'épithélium anal, les néoplasies de la région de la tête et du cou, de préférence de l'oropharynx, ou les néoplasies des amygdales.

7. Agent de déméthylation d'ADN pour une utilisation selon l'une quelconque des revendications 1 à 6, ledit agent de déméthylation d'ADN étant pour une application topique.

8. Agent de déméthylation d'ADN pour une utilisation selon l'une quelconque des revendications 1 à 7, lesdites cellules T spécifiques à un antigène de PV étant des cellules T CD-8+ spécifiques de l'antigène tardif de PV.

9. Agent de déméthylation d'ADN pour une utilisation selon l'une quelconque des revendications 1 à 8, ladite amélioration de la réponse immunitaire étant l'augmentation de la fréquence de cellules T CD-8+ spécifiques de l'antigène tardif de PV activées dans le sang dudit sujet.

10. Agent de déméthylation d'ADN pour une utilisation selon la revendication 9, lesdites cellules T CD-8+ spécifiques de l'antigène tardif de PV activées étant des cellules T CD-8+ spécifiques de l'antigène tardif de PV L1 activées et/ou des cellules T CD-8+ spécifiques de l'antigène tardif de PV L2 activées.

11. Composition pharmaceutique comprenant un agent de déméthylation d'ADN et au moins un support pharmaceutique pour une utilisation dans l'augmentation de la réponse immunitaire à un papillomavirus (PV) d'un sujet infecté par ledit PV, ladite amélioration de la réponse immunitaire étant une augmentation de la fréquence des cellules T CD-8+ et/ou CD4+ spécifiques de l'antigène tardif de PV dans le sang dudit sujet.

12. Composition pharmaceutique pour une utilisation selon la revendication 11, l'agent de déméthylation d'ADN étant présent en une concentration de 0,001 à 50 %.

13. Utilisation *in vitro* d'un agent de déméthylation pour une augmentation de la fréquence des cellules T CD-8+ et/ou CD4+ spécifiques de l'antigène tardif de PV dans un échantillon de sang.

14. Procédé *in vitro* de destruction d'une cellule infectée par un PV, comprenant
la mise en contact de ladite cellule infectée par un PV avec un agent de déméthylation d'ADN,
la mise en contact de ladite cellule infectée par un PV avec des cellules T CD-8+ et/ou CD4+ spécifiques de l'antigène tardif de PV, et
ainsi, la destruction de ladite cellule infectée par un PV.
